# DEMANDE DE BREVET EUROPEEN

(11) **EP 2 380 658 A2**
(43) Date de publication de la demande: **26.10.2011**
(21) Numéro de dépôt: 11290175.6
(22) Date de dépôt: 06.04.2011
(51) Int. Cl.: B01J 13/02, A23P 1/04, A61K 8/11, A61K 9/50, C11D 3/50

(54) **Préparations facilitées de vésicules à l'aide des poly-pentosides d'alkyles et utilisation desdites préparations**

(30) Priorité: 23.04.2010 FR 1001755
(71) Demandeur: Agro Industrie Recherches Et Developpements A.R.D., 51110 Pomacle (FR)
(72) Inventeur: Ernenwein, Cédric, 02860 Nouvion le Vineux (FR); Tranchant, Jean-François, 45760 Marigny les Usages (FR); Pouget, Thierry, 45590 Saint Cyr en Val (FR); Estrine, Boris, 51480 Nanteuil la Forêt (FR); Marinkovic, Sinisa, 08300 Avançons (FR)
(74) Mandataire: Eidelsberg, Victor Albert

(57) **Abrégé**

Système tensioactif apte à former une dispersion de vésicules ou liposomes constitué de polyglycoside d'alkyle de formule RO(X), dans laquelle R est une chaîne alkyle linéaire ou ramifiée, avec ou sans insaturation, et comportant 8 à 12 atomes de carbone, X étant le reste d'un pentose, et n représentant le degré moyen d'oligomérisation est compris entre 1 et 3, et d'un hydrotrope ou co-solvant.

## Description

La présente invention a pour objet un procédé de préparation d'une dispersion de vésicules ou liposomes à l'aide d'au moins un poly-pentosides d'alkyle. Ces systèmes organisés pourront être employés dans diverses industries, telles que la cosmétique, la pharmacie, l'agrochimie, la détergence, le traitement des eaux ou la dépollution des sols et des aquifères.

On entend par systèmes organisés, vésicules ou liposomes, des agrégats supra moléculaires de une ou plusieurs couches de tensioactifs et formant une capsule plus ou moins sphérique de taille micronique ou sub-micronique (généralement de 0,1 à 10 µm) Ces capsules peuvent contenir dans les couches, entre les couches et dans leurs coeurs une phase aqueuse ou non aqueuse pouvant contenir une ou plusieurs matières actives solubilisées ou dispersées. L'ensemble constituant la ou les phases encapsulées. Ce type d'agrégat est aussi communément nommé phase oignon, vésicules multilamellaires ou MLV quand il est constitué d'un empilement de plusieurs couches de tensioactifs du centre vers la périphérie et vésicules unilamellaires ou ULV ou encore liposome quand il est constitué de une ou quelques couches seulement et présentant une cavité au centre servant de lieu privilégié pour l'encapsulation. Il existe de nombreuses méthodes pour la préparation de systèmes organisés, vésicules ou liposomes, notamment décrites par R.G Laughlin dans Colloids Surfaces A : Physicochem. Eng. Aspects 128 (1997). La première méthode consiste à appliquer un cisaillement important à une solution de tensioactifs ou de lipides (généralement sous la forme d'une phase cristal liquide lamellaire). Ce cisaillement peut être imposé par sonication ou en forçant le liquide à passer à travers des orifices calibrés, soit aussi par filtration sur membrane ou encore à l'aide d'un homogénéisateur haute pression. Une autre méthode consiste à précipiter les vésicules par dilution d'une solution de tensioactif dans un solvant approprié ou encore par dialyse. Cette méthode peut être couplée à un cisaillement permettant de contrôler la répartition granulométrique et la taille moyenne des vésicules. La troisième procédure consiste à hydrater un film solide généralement lipidique formé après évaporation d'un solvant. D'autres méthodes mettent en oeuvre des injections de solvants dans des solutions ou le dépôt de gouttelettes de tensioactifs non solubles sur des surfaces polaires et immersion dans l'eau. Enfin les dernières méthodes usuelles sont des réactions chimiques, telles que la précipitation d'acides gras par acidification du milieu ou des réactions de polymérisation.

Le WO 93/19735 décrit une méthode de préparation de capsules de taille contrôlée en fonction du cisaillement imposé par une cellule de type Couette à partir d'une solution concentrée de tensioactifs lamellaires. Le WO 01/32146 décrit des capsules composées de copolymères amphiphiles stabilisés par polymérisation des groupements terminaux, par exemple acrylates. Une méthode de préparation de liposomes par concentration, séchage, réhydratation du film solide, puis filtration est notamment décrite dans le document US 2004057988A1. Le EP 023126A1 donne une méthode de préparation de liposomes par l'action d'un refroidissement rapide, généralement sous azote liquide, d'une dispersion lipidique. Les documents US 4752425 et US 4737323 donnent des méthodes de préparation de liposomes par injection d'une solution lipidique diluée dans un solvant dans une phase aqueuse, puis par une élimination en continue du solvant.

S. Segota dans Advances in Colloid and Interface Science 121 (2006) décrit des structures des tensioactifs particulièrement aptes à former des vésicules et liposomes. La principale classe de molécule possède deux ou plusieurs chaînes lipophiles pour une tête polaire. Principalement ce sont des phospholipides et des glycolipides, qui peuvent être extraits naturellement ou synthétiques. On retrouve aussi des descriptions de vésicules à l'aide de tensioactifs à doubles chaînes comme les hydroxydes de dialkyle-diméthyl-ammonium ou des couples de tensioactifs de charges opposées généralement nommés catanioniques comme les mélanges de dodécyl benzène sulfonate de sodium (anionique) et de bromure de didodécyldiméthyl ammonium (cationique).

Des vésicules ont aussi été observées avec des tensioactifs à simple chaîne mais généralement dans des mélanges complexes tensioactifs/co-tensioactifs/eau comme par exemple des mélanges d'alcools gras éthoxylés (comme l'alcool laurique à 5 motifs éthoxyléniques ou C12E5) avec une incorporation de cholestérol comme co-tensioactif absolument nécessaire à la formation des vésicules.

Les polyglycosides d'alkyle constituent une classe de tensioactifs non ioniques particulièrement appréciés en raison de l'origine renouvelable des matières premières qui les constituent, de leur relative douceur pour la peau et les muqueuses, de leur facilité de biodégradation et de leur faible impact environnemental.

Le DE 19634374A1 décrit des dispersions de vésicules formées avec des polyglucosides d'alkyle ou APG de formule :

RO(G)n (1)

dans laquelle R est un radical aliphatique linéaire ayant de 12 à 22 atomes de carbone et n représente le degré de polymérisation des sucres ou Dp et est compris entre 1 et 2 (produit A). L'APG est obligatoirement en mélange avec un alcool gras linéaire de 12 à 22 atomes de carbone (produit B) dans un rapport massique A/B de 1/ (0,1 à 2). Autrement dit, l'alcool C12 à C22 représente de 9,1 à 66,7% en poids du poids total du système A+B. Préférentiellement, les mélanges contiennent aussi des co-tensioactifs lipidiques, tels que des stérols comme les phytostérols ou des dicétylphosphates, eux-mêmes connus pour former facilement des liposomes.

D. Balzer dans Nonionic Surfactants, Alkyl Polyglucosides, Surfactant Science series vol. 91, M. Dekker, NY, 2000 a étudié les structures cristallines et cristal-liquides des APG en solution. Les APG possédant des chaînes alkyles ayant 8 à 10 atomes de carbone forment des phases lamellaires dans une plage de concentration massique allant de 78 à 81% (C8/C10 APG n=1,5). Les APG plus longs, possédant des chaînes alkyles ayant 12 à 14 atomes de carbone forment quant à eux des phases lamellaires à une concentration au moins supérieure à 65% massique (C12/C14 APG n=1,3).

Considérant que la formation d'une phase lamellaire constitue une condition nécessaire à la formation de vésicules ou liposomes, les APG de formule (1) ne pourront donc former de tels objets qu'à des concentrations au moins supérieures à 65%.

Enfin le US 6,251,425 B de 2001 démontre clairement que les APG sont des tensioactifs primaires qui ne peuvent former de vésicules en absence de stéroïde.

La complexité de fabrication et de mise en oeuvre des vésicules et liposomes d'une part et la difficulté pour obtenir facilement de tels systèmes organisés, stables et diluables avec des APG uniquement constituent des défauts techniques que la présente invention souhaite corriger.

Les vésicules ou liposomes de la présente invention sont préparés avec au moins un polyglycoside d'alkyle de formule :

RO(X)n (2)

dans laquelle R est un radical aliphatique linéaire ou ramifié, avec ou sans insaturation ayant 8 à 10 atomes de carbone. X est un reste de xylose sous sa forme isomérique alpha ou bêta, de la série L ou D et sous sa forme furanosique ou pyranosique, n représente le degré d'oligomérisation moyen et est généralement compris entre 1 et 3, de préférence compris entre 1 et 1,5.

Il a été trouvé, et ceci constitue le fondement de la présente invention, que les polyglycosides d'alkyle de formule (2) peuvent former facilement une dispersion de systèmes organisés, vésicules ou liposomes stables et diluables et ce même avec des longueurs de chaînes alkyle linéaires et inférieures ou égales à 12 atomes de carbone. On entend par obtenir "facilement" des systèmes organisés, vésicules ou liposomes, l'absence de co-tensioactifs, notamment de stéroïdes formant à eux seuls de tels systèmes ou d'alcools gras, la surprenante facilité de mise en oeuvre sans l'utilisation de mélangeurs particuliers, comme par exemple des appareils de sonication ou des mélangeurs de type couette, sans l'obligation de passer par une phase solide obtenue par précipitation ou congélation, ni par l'injection de solvant ou l'évaporation de solvant. On entend par "stable", la persistante et le maintien de la taille moyenne des vésicules ou liposomes formés selon la méthode de l'invention en fonction de la durée de stockage et de la température de stockage et généralement comprise entre 4°C et 60°C. On entend par "diluable", la possibilité de disperser une solution concentrée de vésicules ou liposomes dans un solvant approprié, généralement aqueux, et ce par un facteur de dilution de 2 à 1000 fois, et de préférence de 5 à 100 fois, tout en assurant la persistance et la taille des objets initiaux. L'une des caractéristiques de l'invention est la surprenante stabilité des systèmes organisés, vésicules ou liposomes formés et ce même dans des formulations complexes, telles que par exemple des émulsions, des préparations lavantes ou détergentes, des formulations phytosanitaires ou des préparations pour la dépollution des sols.

L'invention a donc pour objet un procédé de préparation d'une dispersion de vésicules multilamellaires ou liposomes comprenant la dispersion d'un système tensioactif apte à former des vésicules ou liposomes dans une phase aqueuse, un solvant ou une huile, caractérisé en ce que ce système tensioactif contient en poids de 5 à 9%, de préférence de 70 à 90%, au moins un polyglycoside d'alkyle de formule RO(X)n, dans laquelle R est une chaîne alkyle linéaire ou ramifiée, avec ou sans insaturation, et comportant 8 à 10 atomes de carbone, X est le reste de xylose, et n représente le degré moyen d'oligomérisation et est compris entre 1 et 3.

L'invention a aussi pour objet un procédé d'inclusion d'une ou plusieurs matières actives cosmétiques, pharmaceutiques, phytopharmaceutiques, de parfums, d'enzymes, de nutriments, d'oligo-éléments, de matériaux biologiques, d'huiles essentielles, d'agents aptes à oxyder ou dégrader des molécules organiques, dans des vésicules multilamellaires ou liposomes, consistant à mélanger lesdites matières actives brutes ou diluées indifféremment dans une huile, un solvant ou dans l'eau avec un système tensioactif puis à disperser ce mélange dans une phase aqueuse, un solvant ou une huile, caractérisé en ce que ce système tensioactif contient en poids de 5 à 9%, de préférence de 70 à 90%, d'au moins un polyglycoside d'alkyle de formule RO(X)n, dans laquelle R est une chaîne alkyle linéaire ou ramifiée, avec ou sans insaturation, et comportant 8 à 10 atomes de carbone, X est le reste de xylose, et n représente le degré moyen d'oligomérisation et est compris entre 1 et 3.

Le terme "dispersion" comprend indifféremment la dissolution, la solubilisation, la formation d'une suspension ou d'une solution colloïdale dans une phase continue aqueuse ou non aqueuse.

Le système tensioactif suivant l'invention peut être constitué en poids :
- de 5 à 99%, de préférence de 30 à 90%, de polyglycoside d'alkyle de formule RO(X)n, dans laquelle R est une chaîne alkyle linéaire ou ramifiée, avec ou sans insaturation, et comportant 8 à 10 atomes de carbone, X étant le reste xylose, et n représentant le degré moyen d'oligomérisation est compris entre 1 et 3, de préférence entre 1 et 1,5 ;
- de 0,1 à 70% d'un ou plusieurs co-tensioactifs ayant une HLB au moins égale à 10 ;
- de 0 à 70%, de préférence de 0,1 à 70%, d'un hydrotrope ou co-solvant choisi parmi les alcools linéaires ou ramifiés de C1 à C20, les éthers de glycols, les glycosides d'amyle, de butyle, d'hexyle, de 2-éthyl-hexyle, d'octyle, d'iso-nonyle, d'iso-décyle, d'octyle-décyle, le glycérol et les dérivés partiels de glycérol, les terpinéols, les esters d'acides organiques comme l'acide acétique, formique, lactique, succinique, tartrique, glutarique, glutamique, adipique, laurique, oléique ; et
- de 0,1 à 70% d'eau.

Le système peut contenir en poids de 95 à 5%, de préférence de 70 à 10%, d'un autre polyglycoside d'alkyle, de préférence d'un pentoside, mais aussi d'un hexoside.

Afin de garantir une meilleure stabilité et une meilleure dispersion des vésicules ou liposomes, on peut utiliser de manière avantageuse un système tensioactif comportant un ou plusieurs polyglycosides d'alkyle de formule (2) et un ou plusieurs co-tensioactifs et/ou un ou plusieurs co-solvants. Le ou les co-tensioactifs ne formant pas eux-mêmes nécessairement des systèmes organisés ou liposomes. Le rapport massique polyglycoside d'alkyle sur co-tensioactif et/ou co-solvant sera alors compris entre 0,5 et 1 000, de préférence de 1 à 100. Autrement dit, de préférence le co-tensioactif ou co-solvant représentera de 1 à 50% en poids par rapport au poids total.

De préférence, pour obtenir une dispersion stable et diluable en milieu aqueux, on utilisera un co-tensioactif avec une HLB supérieure à 10, de préférence encore une HLB supérieure à 15. La HLB s'entend de la Balance-Hydrophile-Lipophile notamment décrite dans "Agents de surface et émulsion" dans la collection Galenica aux éditions "Technique et Documentation (Lavoisier)" de 1983 et peut être obtenus par calcul selon la méthode de Griffin (Griffin WC: "Calculation of HLB Values of NonIonic Surfactants, "Journal of the Society of Cosmetic Chemists 5 (1954): 259) ou par la méthode de Davies (Davies JT: "A quantitative kinetic theory of emulsion type, I. Physical chemistry of the emulsifying agent," Gas/Liquid and Liquid/Liquid Interface. Proceedings of the International Congress of Surface Activity (1957): 426-438).

On utilisera par exemple et sans l'intention de s'y limiter comme co-tensioactif des polyglycosides d'octyle, d'hexyle, de 2-éthyl-hexyle, de butyle, de 2 méthyl-butyle ou de 3 méthyl-butyle, des esters de sorbitan polyéthoxylés comme les esters de sorbitan éthoxylés d'acide laurique, oléique, stéarique, tri-oléique, des huiles polyéthoxylées comme l'huile de ricin hydrogénée à 40 mole d'oxyde d'éthylène, des alcools gras fortement éthoxylés comme l'alcool laurique à 23 motifs éthoxylés, l'alcool oléique à 28 motifs éthoxylés. Au titre de co-solvants, peuvent aussi être utilisés des alcools linéaires comme l'éthanol, le butanol, le pentanol, l'hexanol, l'octanol, le décanol, le dodécanol ou les alcools ramifiés comme le 2-éthyl-hexanol, l'alcool isoamylique, le 3- méthyl-butanol, le 2-butyl-octanol, le 2-butyl-décanol, le 2-hexyl-octanol, le 2-hexyl-décanol, le 2-octyl-décanol, le 2-hexyl-dodécanol, le 2-octyl-dodécanol, le 2-décyl-tétradécanol, les terpinéols ainsi que le glycérol ou ses dérivés dont les esters de mono-glycérol, les polyglycérols et ses dérivés comme le mono-stéarate de polyglycérol ou le polyricinoléate de polyglycérol, le butylène glycol, le propylène glycol, les éthers de glycols comme le di-éthylène glycol butyl-éther, le di-éthylène glycol propyl-éther, le tri-éthylène glycol éthyl-éther, le tri-éthylène glycol méthyl-éther le tri-éthylène glycol butyl-éther et leurs acétates, peuvent aussi être utilisés des esters d'acides gras comme le esters méthylique, butylique, isobutylique, amylique, propylique, iso-propylique, 2-éthyl-hexylique, héxylique, octylique, décylique, isodécylique d'acides gras de C12 à C24 saturés ou ayant une ou plusieurs insaturations.

De préférence, pour obtenir une dispersion stable et diluable en milieu non aqueux, on utilisera avantageusement un co-tensioactif d'HLB inférieure à 10 ou préférentiellement inférieure à 7, préférentiellement encore inférieure à 5, au lieu d'un co-tensioactif d'HLB au moins égale à 10. A titre d'exemple et sans l'intention de s'y limiter, nous pouvons choisir comme co-tensioactif d'HLB bas, les esters de sorbitan comme le monolaurate, le mono-oléate, le mono-stéarate, le trioléate, le tristéarate de sorbitan, les esters de sorbitan faiblement éthoxylés comme le POE(2) stéarate de sorbitan, les esters de propylène-glycol d'acides gras comme le mono-oléate de propylène glycol, le propylène glycol monomyristate, les monoester de glycérol comme le mono-oléate de glycérol, le mono-stéarate de glycérol, les saccharo-esters comme le myristate, palmitate ou stéarate de saccharose, les di, tri et polystéarate de saccharose, les di, tri et polypalmitate de saccharose, les di, tri et polylaurate de saccharose, les alcools gras faiblement éthoxylés comme l'alcool cétylique POE(2), l'alcool oléique POE(2), les huiles faiblement éthoxylées comme l'huile de ricin hydrogénée à 5 moles d'oxyde d'éthylène.

L'invention trouve des applications dans de nombreux domaines. Elle vise des vésicules ou liposomes qui contiennent de 1 à 99% en poids par rapport au poids total des vésicules ou liposomes, de préférence de 10 à 95% et de préférence encore de 30 à 90%, d'une ou plusieurs matières actives cosmétiques, pharmaceutiques, phytopharmaceutiques, de parfums, d'enzymes, de nutriments, d'oligo-éléments, de matériaux biologiques, d'huiles essentielles, d'agents aptes à oxyder ou dégrader des molécules organiques, brutes ou diluées indifféremment dans une huile, un solvant ou dans l'eau. L'invention vise aussi une émulsion huile dans eau, solution aqueuse ou hydro-alcoolique, gel aqueux ou hydro-alcoolique contenant de 0,01 à 90% en poids, de préférence de 0,1 à 50% et de préférence encore de 1 à 20% en poids de vésicules ou liposomes et préparés à l'aide d'au moins un système tensioactif suivant l'invention, ainsi qu'une émulsion eau dans huile, solvants ou mélanges de solvants, microémulsion, contenant de 0,01 à 90% en poids, de préférence de 0,1 à 50% en poids et de préférence encore de 1 à 10% en poids de vésicules ou liposomes et préparés à l'aide d'au moins un système tensioactif suivant l'invention.

### La cosmétique et la (dermo)pharmacie

L'invention sera utilisée dans le domaine de la cosmétique, de la dermopharmacie et la pharmacie afin d'encapsuler des matières actives pour notamment les libérer au cours du temps ou en fonction de contraintes externes comme l'élévation de la température, l'application d'un cisaillement ou des réactions chimiques comme des modifications de pH, des réactions d'hydrolyse, d'oxydation ou des réactions enzymatiques. On peut aussi rechercher à encapsuler des matières sensibles, notamment de l'oxydation ou de l'exposition aux rayons UV, dans le but de les protéger et maintenir leur efficacité. On peut aussi rechercher à véhiculer les vésicules vers des sites d'action ciblée à travers le derme, l'épiderme ou l'hypoderme ou les absorber notamment sur la peau ou les cheveux afin de prolonger l'effet recherché.

L'une des particularités de l'invention est l'obtention de vésicules stables même dans des environnements complexes, notamment dans les émulsions et les solutions concentrées en tensioactifs.

Les vésicules préparées par les méthodes de l'invention seront donc particulièrement utilisées dans des préparations aqueuses sous la forme de solutions, de dispersions, de gels, de lotions et en émulsions huile dans eau (H/E), eau dans huile (E/H) ou des systèmes complexes tels que les émulsions multiples (E/H/E ou H/E/H), notamment dans des préparations comme des crèmes, des laits, des pommades, des onguents, des gels, des lotions.

Comme les vésicules préparées selon les méthodes de la présente demande peuvent être aussi bien dispersées en milieu aqueux que non aqueux, elles peuvent aussi trouver des applications dans des domaines ne contenant pas ou très peu d'eau. Citons par exemple la formulation d'huile de massage, les huiles de protection solaire, les huiles pour les sportifs ou pour le traitement de la peau. L'invention trouvera aussi des applications dans des préparations solides ou pâteuses comme dans les maquillages, tels que les rouges à lèvres, les mascaras ou les fonds de teint.

Les matières encapsulées peuvent être par exemple des vitamines, comme la vitamine A, E ou C, des parfums synthétiques ou naturels comme l'acétate de benzyle, l'acétate de linalyle, le benzoate de linalyle, le citral, le citronellal, le lilial, l'eugénol, le citronelol, le linalool, les dérivés terpéniques, les essences de sauge, de camomille, d'oeillet, de vétiver, de lavandin, des huiles essentielles comme les huiles essentielles de lavande, thym, sarriette, sauge, menthe, cumin, carvi, anis vert, fenouil, aneth, eucalyptus, cajeput, niaouli, girofle, pin, cèdre, cyprès, genévrier, citron, orange, bergamote, cannelle, laurier, camomille, des anti-inflammatoires comme des extraits de plantes, l'alpha-bisabolol, le panthénol, l'alpha-tocophérol, des anti-brûlures comme l'allantoïne, des anti-âges comme le rétinol, des agents autobronzants comme la dihydroxyacétone (DHA), des agents dépigmentants comme l'acide kojique, l'acide coumarique, l'arbutine, des actifs amincissants comme la caféine, des agents anti-transpirants comme des sels d'aluminium, de zinc ou de zirconium, l'acide pentaacétique de diéthylène triamine, des agents antipelliculaires comme la pyrithione de zinc ou d'aluminium, l'acide salycylique, des sels de pyridone et des dérivés des pipérazine, les filtres UV comme les dérivés de la benzophénone, les esters d'acides cinnamiques, les esters d'acide salicylique, le 3-benzylidène camphre, les antioxydants comme l'acide ascorbique et ses dérivés, l'acide citrique et ses dérivés, l'acide glutamique, les glutamates et ses dérivés, l'acide lactique et ses dérivés, l'acide tartrique et ses dérivés, les bioflavonoïdes, le buthylhydroxy-hydroxyanisol, le carotène et ses dérivés, les sulfites comme les bisulfites de sodium, le chlorobutanol, les agents conservateurs comme les parabens, le phénoxyéthanol, le 2-bromo-2-nitropropane-1,3-diol, les formaldéhydes, le pantanédiol, l'acide sorbique, les agents hydratants comme le glycérol, le sorbitol, le collagène, le pro-collagène, la gélatine, l'aloe-vera, l'acide hyaluronique, l'urée, les agents répulsifs pour les insectes comme l'acétamipiride, l'étofenprox, le permethrin, la cypermethrin, la N,N-diéthyl-m-toluamide, le butylacétylaminopropionate, les actifs pharmaceutiques, comme les désinfectants tels que le gluconate de chlorexidrine, le chlorure de benzalkonium, l'acide benzoique, le chlorure de cétylpyridinium, les anti-inflammatoires comme la teinture d'arnica, l'eucalyptol, le menthol, le diméthoxy-1,2-benzène, les antiacnéiques comme les dérivés de la trétinoïde, l'acide azélaïque, l'acide salicylique, les antifongiques comme les dérivés de la pyridone tel que le cyclopiroxolamine, les dérivés d'imidazole tels que le clotrimazole, l'acide folique, la riboflavine, les séquestrants comme l'acide mucique, l'acide phytique, le NTA, l'EDTA, les colorants, les ajusteurs de pH, des arômes naturels ou de synthèses.

### Détergence

Les systèmes organisés, vésicules ou liposomes préparés selon les méthodes dé la présente invention seront utilisés dans des formulations détergentes notamment dans les lessives, les adoucissants, les produits de nettoyage des surfaces dures, les produits pour le lavage de la vaisselle ou les produits de nettoyage des véhicules.

Dans le domaine des lessives, notamment des lessives liquides ou les gels, les matières encapsulées peuvent être, par exemple, des parfums notamment pour prolonger l'effet "frais" et l'odeur plaisante du linge propre ou pour les protéger des enzymes présentes dans la formulation notamment des lipases qui peuvent dénaturer ce dernier durant le cycle de lavage, les enzymes peuvent aussi être encapsulées afin de les protéger des rayonnements UV, des dégradations thermiques ou chimiques notamment dues aux pH alcalins. Les vésicules peuvent donc aussi contenir des agents absorbant les UV, des agents blanchissants ou azurants, ainsi que des activateurs de blanchiment, des vitamines non ioniques ou cationiques, des agents anti-microbiens, anti-fongiques ou anti-viraux, des agents anti-transpirants, des déodorants, des agents nutritifs, des agents de protection de la peau et des hydratants de la peau.

Au titre des parfums et agents odorants, on peux citer par exemple et sans l'intention de s'y limiter, des parfums synthétiques ou naturels comme l'acétate de benzyle, l'acétate de linalyle, le benzoate de linalyle, le citral, le citronellal, le lilial, l'eugénol, le citronelol, le linalool, les dérivés terpéniques, les essences de sauge, de camomille, d'oeillet, de vétiver, de lavandin, des huiles essentielles comme les huiles essentielles de lavande, thym, sarriette, sauge, menthe, cumin, carvi, anis vert, fenouil, aneth, eucalyptus, cajeput, niaouli, girofle, pin, cèdre, cyprès, genévrier, citron, orange, bergamote, cannelle, laurier, camomille.

Au titre d'agent blanchissant, on peut utiliser par exemple le péroxyde d'hydrogène, l'hypochlorite de soude, les percarbonates de potassium, les perborates de sodium ou les péracides

Au titre des activateurs de blanchiment, nous citerons par exemple la N,N,N',N'-tetraacétyle éthylène diamine ou ses dérivés, le nonanoyloxybenzène sulfonate de sodium et ses dérivés ou encore le 4-benzoyloxy-benzène sulfonate de sodium.

Les enzymes permettent de compléter l'action lavante des préparations, notamment sur les salissures grasses et alimentaires, nous pouvons par exemple utiliser des amylases, des cellulases, des lipases, des péroxydases, des protéases commercialisées notamment par la société Novosyme.

### Formulation Phytosanitaire

L'invention trouve aussi des applications dans le domaine de l'agrochimie notamment pour la formulation des herbicides, insecticides, fongicides, algicides, rodenticides, molluscicides, acaricides, des régulateurs de croissance, des nutriments, des oligo-éléments, des fertilisants, des éliciteurs, des répulsifs contre les insectes, les mammifères ou les oiseaux, des appâts pour les insectes ou les rongeurs. Les formulations phytosanitaires peuvent être sous la forme de solutions aqueuses ou non aqueuses, d'émulsions huile dans eau (H/E) ou eau dans huile (E/H), de microémulsions, de suspoémulsions, de gels, de concentrés émulsionnables, de granulés, de poudres mouillables, de tablettes ou bâtonnets fragmentables ou dispersables.

L'objectif d'encapsuler une matière dans des vésicules de l'invention est de permettre un dosage précis et une libération contrôlée des actifs, de réduire la toxicité des pesticides ou de protéger les matières biologiquement actives notamment des contraintes extérieures comme les rayonnements UV, des réactions chimiques telles que celles induites par des modifications de pH ou de compléxation avec des ions notamment du calcium ou du magnésium présent dans l'eau dure.

Au titre des pesticides pouvant être encapsulés par les vésicules de l'invention, citons par exemple et sans l'intention de s'y limiter, les herbicides de type amide comme le benzipram, le cyprazole, le formesafen, les anilides comme le métamifop, le pentanochlor, les arylalanines, les chloroacétanilides comme l'alchlor, le métazochlor, le propachlor, le terbuchlor, les sulfonanilides comme le benzofluor, le pyrimisulfan, les acides benzoïques comme le dicamba, le 2,3,6 TBA , le tricamba, les acides phtaliques, les acides picoliniques, les acides quinoline-carboxyliques, les benzoylcyclohexanédiones, les benzofuranyl-alkylsulfonates, les carbamates, les carbanilates comme le chlorobufam le chloropropham, le desmedipham, le phenmedipham, le phenmedipham-éthyl, les cyclohexène oximes comme cloproxydim, cycloxydim, profoxydim, les cycloproxylisoxazoles, les dicarboximidines comme le benzfendizone, le flumixazin, les dinitroanilines, les dinitrophenoles, les diphényl-éthers comme l'éthoxyfen, les dithiocarbamates, les herbicides aliphatiques halogénés, les imidazolinones, les nitriles comme le bromoxynil, lé chloroxynil, le dichlobenil, les herbicides organophosphorés comme le 2,4 DEP, la fosamine, le glufosinate, le glyphosate, les oxadiazolonz, les herbicides phénoxy comme le 2,4 DEB, l'etnipromid, le 2,4D, le MCPA, le 2,4 DB, le dichloprop, le mecoprop, le diclofop, le fenaxoprop, le fluazifop, le clodinafop, l'haloxifiop, les phénylènediamines, les pyrazoles comme le metazochlor, le benzofenap, les pyridazines, les pyridazinones, les pyridines comme l'aminopyralid, les pyrimidinédiamines, les thiocarbamates comme le sulfallate, le thiobencarb, les thiocarbonates, les triazines comme le trihydroxytriazine, l'atrazine, la cyprazine, l'atraton, le prometon, l'aziprotryne, les triazinones, les triazoles, les triazolones comme le bencarbazone, la propoxycarbazone, les triazolopyrimidines, les herbicides à base d'urée comme les phénylurées, les sulfonylurés tels que l'amidosulfuron, l'ethoxysulfuron, le mezosulfuron, le chlorsulfuron, le metsulfuron, le triasulfuron, les thiadiazolylurées tels que le buthiron, le thiazafluron ainsi que les ammoniums quaternaires et leurs dérivés, les herbicides inorganiques comme le sulfate de cuivre, le sulfamate d'ammonium, le chlorate de soude, le cyanate de potassium.

Au titre des insecticides pouvant être encapsulés citons par exemple ceux issus de plantes comme par exemple le d-limonène, la nicotine, les pyréthrines, les jasmolins, les pyréthroïdes, les insecticides carbamates comme les aryl-méthylcarbamates, les mono méthyl ou diméthylcarbamates héterocycliques tels que le carbofuran, le carbosulfan, le primicarb, les oximes carbamates tels que l'oxamyl, le thiofanox, le méthomyl, les insecticides organochlorés comme le DDT, le méthoxychlor, l'hexachlorocyclohexane (HCH) et ses dérivés, l'heptachlor, le mirex, les insecticides organophosphorés comme les orthophosphates (chlorfenvinphos, dichlorvos), les phosphorothionates (bromphos, diazinon, parathion), les phosphorothiolates, les phosphorothiolothionates (diméthoate, disulfoton, menazon), les phosphonates (triclorphon, butonate), les pyrophophoramides (shradan), les insecticides bloquant la croissance des insectes comme les inhibiteurs de chitin tels que le novaluron, le penfluron, les hormones juvéniles naturelles ou synthétiques comme la méthoprène, la kinoprène, les insecticides formamidines comme le chlorodimeform, l'amitraz, les insecticides dérivés de benzoylphénylurée comme le diflubenzuron, le penfluron, les insecticides inorganiques comme le borax, l'oléate de cuivre, le thiocyanate de sodium.

Au titre des fongicides pouvant être encapsulés selon l'invention citons par exemple les fongicides cuivrés, tels que la bouillie bordelaise, l'oxycloride de cuivre, les oxydes cuivrés, les hydroxides de cuivre, les fongicides à base de mercure comme les organomercurés tels que le PMA, l'acétate de méthoxyéthylmercure, le thiomersal, les dithiocarbamates comme le thiram, le maneb, les phtalimides comme le captan, les phthalonitriles comme le chlorothalonil, les dicarboximides comme le vinclozolin, les dinitrophenol comme le dinocap, le dinocton, les benzimidazoles comme le benomyl, le cypendazole, les oxathiines comme le carboxin, les morpholines comme le tridemorph, le carbamorph, les hydroxyaminopyrimidines comme l'ethirimol, les fongicides antibiotiques tels que la kazugamycin, les strobillurines et leurs dérivés, les phénylamides comme le metalaxyl, les composés organophosphorés comme le pyrazophos, le fosetyl, les imidazoles comme le cyazofamid, l'irpodione, le prochloraz, les triazoles comme l'hexaconazole, l'epxiconazole, des dérivés de la guanidine, les aromatiques chlorés comme la quintozene, le dichloran, la chlorothalonil, le dicloran.

### Application pour le traitement des eaux et la dépollution des sols et des aquifères

L'invention trouve aussi des applications dans le domaine de la dépollution des sols des aquifères et le traitement des eaux, notamment dans les fosses septiques.

Il est déjà connu que l'incorporation d'agent permettant l'oxygénation du milieu permettait d'augmenter la performance des opérations de bioremédiation des sols pollués, notamment par des hydrocarbures. L'objectif est d'augmenter le nombre de bactéries capables de biodégrader les polluants présents par injection de formulations capables, généralement par des réactions chimiques, de fournir de l'oxygène au milieu. Ce type de préparation est notamment décrit dans le document US 5,264,018.

Dans le cas de pollution des aquifères et des nappes phréatiques, notamment par des polluants chlorés, il peut être intéressant d'incorporer des agents chimiques permettant d'amorcer des réactions d'oxydation, soit pour dégrader complètement et directement le polluant, soit pour rendre le polluant plus facilement accessible aux micro-organismes pour sa complète biodégradation. Ces types de composés sont par exemple des réactifs de Fenton, tels que ceux décrits dans le US 6,268,205. Néanmoins dans tous les cas, les réactifs peuvent être relativement instables, peuvent se dégrader rapidement et perdre en grande partie leur efficacité avant d'atteindre leur site d'action. Il est donc intéressant d'encapsuler dans des vésicules préparées selon l'invention, les réactifs permettant de libérer de l'oxygène et/ou de l'hydrogène et/ou d'oxyder les polluants. Ceci afin de garantir leur transport vers la zone à traiter, de permettre une libération contrôlée pour maintenir une efficacité à long terme ou de réduire la toxicité desdites matières pour le milieu ambiant.

A titre d'exemple et sans l'intention de s'y limiter, les matières pouvant être encapsulées par une dispersion de vésicule de l'invention seront le péroxyde d'hydrogène, des complexes urée/péroxyde d'hydrogène, du percarbonate de sodium, du péroxyde de calcium, du péroxyde de magnésium, des permanganates de potassium, du persulfate de sodium, du bisulfite de sodium. Des catalyseurs, des nutriments riches en oligo-éléments , des enzymes, des bactéries, des ajusteurs de pH, des complexants pourront être aussi encapsulés afin de potentialiser l'action d'oxygénation, de bioremédiation ou d'oxydation du milieu traité.

### Préparation

De préférence les matières à encapsuler sont liquides ou solubilisées dans un liquide. Elles peuvent donc être diluées dans l'eau, un solvant ou une huile à la concentration permettant leur complète solubilisation. Les matières actives et éventuellement leur solvant constituent ainsi la phase à encapsuler.

La phase à encapsuler représente de 1 à 99% en poids par rapport au poids total des vésicules ou liposomes, de préférence de 10 à 95% et de préférence encore de 30 à 90%.

Les vésicules ou liposomes seront préparées en mélangeant intimement la phase à encapsuler avec le système tensioactif suivant l'invention à la température ambiante ou à une température supérieure mais de préférence inférieure à 100°C. On choisira de préférence des mélangeurs à bas cisaillements comme des agitateurs pendulaires munis d'une ou plusieurs hélices ou d'une ancre, des agitateurs à double rotations munis d'une agitation centrale à une ou plusieurs hélices et d'une agitation périphérique munis d'une ou plusieurs pâles raclantes épousant la forme du réacteur. On pourra aussi utiliser des pétrins et malaxeurs. On proscrira de préférence les agitateurs à hauts cisaillements comme les rotor-stators, les hélices crantées tournant à haute vitesse, les agitateurs horizontaux munis d'un axe et de pales mises en rotation à l'aide d'un moteur, les moulins colloïdaux. La durée d'agitation pourra varier de quelques minutes à plusieurs heures et ce jusqu'à l'obtention d'un liquide visqueux, généralement ayant une viscosité mesurée à l'aide d'un viscosimètre de type Brookfield de 100 à 500 000 centipoises à la température du mélange ou d'une pâte homogène (notamment à 25°C). Cette préparation concentrée en vésicules ou liposomes pourra être utilisée aussitôt pour la préparation de produits pharmaceutiques, cosmétiques, détergents, pour le traitement des eaux ou des aquifères ou être stockée à une température d'usage, généralement de 4 à 45°C ou supérieure mais de préférence inférieure à 100°C. Afin d'augmenter la stabilité du concentré l'ajout d'agents bloquant ou ralentissant la prolifération bactérienne ou fongique peut être nécessaire. Dans ce cas ils seront rajoutés durant la phase de mélange et aux concentrations garantissant leur efficacité.

Dans les préparations cosmétiques constituées par un continuum aqueux, soit sous la forme de solution, de gel ou d'émulsion huile dans eau, le taux de vésicules ou liposomes variera de 0,01 à 50% par rapport au poids total de la préparation, de préférence de 0,1 à 20%. Dans le cas de préparations inverses, notamment d'émulsions eau dans huile, le taux d'incorporation sera généralement de 0,01 à 30% par rapport au poids total et de préférence de 0,1 à 10%.

Dans les préparations détergentes, notamment celles visant à encapsuler une ou plusieurs enzymes dans des lessives liquides ou des gels, les vésicules ou liposomes pourront contenir par rapport à leurs poids de 2 à 15% d'enzymes et seront incorporés à hauteur de 0,1 à 15% en poids par rapport au poids total de la préparation.

Pour l'encapsulation de parfums ou d'huiles essentielles, de manière à prolonger l'effet frais du linge par exemple, le taux d'incorporation des vésicules ou liposomes dans les préparations variera par exemple de 0,1 à 20% par rapport au poids total de la préparation.

Dans le cas des préparations phytosanitaires, la concentration des matières actives dans les vésicules ou liposomes et le taux d'incorporation dans les préparations dépendront de la nature desdites matières actives et des prescriptions réglementaires du pays autorisant leur utilisation. Le taux d'incorporation peut varier par exemple de 0,01 à 10% en poids de la préparation pour des molécules actives très efficaces comme celles de la famille des sulfonyl-urée comme le metsulfuron méthyle, ou de 10 à 90% en poids de la préparation pour des herbicides moins actifs comme par exemple le 2,4D, sel de diméthylamine.

Pour le traitement des eaux et des fosses septiques, l'application d'une préparation contenant de 0,1 à 90% en poids de vésicules ou liposomes, de préférence de 1 à 50%, et de préférence encore de 5 à 30% sera réalisée de manière discontinue. Pour le traitement des aquifères et des nappes phréatiques, des traitements d'une préparation contenant de 0,01 à 50% en poids de vésicules ou liposomes, de préférence de 0,1 à 10% pourra être appliquée de manière discontinue ou continue pendant la durée de l'opération de dépollution.

### Méthode de caractérisation des vésicules et liposomes

La technique de caractérisation utilisée consiste à observer au microscope électronique à transmission des répliques de cryofracture des solutions de tensioactifs. Cette méthode est largement documentée, notamment par A.GULIK, L.P. AGGERBECK, J.C.DEDIEU and T. GULIK-KRZYWICKI, dans Journal of Microscopy, Vol. 125, Pt 2, February 1982, pp. 207-213, ou plus récemment par O. MONDAIN-MONVAL dans Current Opinion in Colloid and Interface Science, 10 (2005), 250-255.

La méthode qui a été utilisée est le cryodécapage qui permet de réaliser une réplique de la structure observable par microscopie électronique à transmission. Cette technique comporte quatre étapes essentielles:
1 la congélation ;
2 la fracture et le "etching" ;
3 l'ombrage et la formation de la réplique ;
4 le nettoyage de la réplique.

Enfin la visualisation de la réplique à l'aide d'un microscope électronique à transmission et l'analyse visuelle des clichés.

Pour être en accord avec l'invention, les clichés doivent démontrer une dispersion homogène d'objets sphériques ou quasi-sphériques en positif et négatif et présentant dans le cas des vésicules multilamellaires plusieurs stries sphériques en périphérie traduisant l'enroulement de plusieurs couches. En outre les clichés ne doivent pas présenter de larges zones de clivages, représentées par des rayures linéaires et traduisant la présence d'une phase lamellaire non enroulée résultant d'un système incapable de former spontanément des vésicules ou liposomes.

L'invention sera illustrée plus en détail par les exemples suivants, donnés uniquement à titre illustratif et dans lesquels les caractéristiques des produits obtenus sont évaluées selon les critères décrits ci-dessus.

### Exemple 1

### Mise en évidence des vésicules multilamellaires selon l'invention

5,25g de poly-xylosides d'octyle/décyle (XYLC8/10 DP=1,13) sont mélangés à 44,75g d'eau/glycérine à l'aide d'un agitateur mécanique à 500 tours/min pendant 30 minutes à 80°C, puis refroidis à température ambiante tout en maintenant l'agitation. Une réplique de cryofracture est ensuite effectuée sur cette préparation et visualisée en microscopie électronique à transmission (MET).

Le cliché obtenu (Cliché 1) montre clairement la présence de nombreuses vésicules de 0,2 à 1µm. De nombreux sillons circulaires sont visibles et traduisent ainsi la formation de vésicules multilamellaires.

### Exemple 2

### Mise en évidence de la robustesse des vésicules multilamellaires selon l'invention

Un poly-xyloside de décyle (Xyl C10) est dispersé dans une solution eau/glycérine à différentes concentrations à l'aide d'un agitateur mécanique à 500 tours/min pendant 30 minutes à 80°C, puis refroidi à température ambiante tout en maintenant l'agitation.

Chaque dispersion est prélevée pour obtenir une réplique de cryofracture qui est ensuite visualisée au MET. La persistance des vésicules multilamellaires est vérifiée par la présence d'objets caractéristiques.

Les résultats obtenus sont repris dans le tableau suivant et par les clichés 2 à 5.

| | | | | |
|---|---|---|---|---|
| Facteur de dilution | 1 | 2 | 8 | 16 |
| % XYLC10 DP=1,5 | 15 | 7,5 | 1,875 | 0,9375 |
| Observation au TEM | Dispersions homogènes de vésicules multilamellaires | Dispersions homogènes de vésicules multilamellaires | Structures unilamellaires en mélange avec quelques multilamellaires | Structures unilamellaires en mélange avec quelques multilamellaires |
| N° Clichés | 2 | 3 | 4 | 5 |

### Exemple Comparatif 1

Des poly-xylosides de décyle (XylC10 DP=1,25) et des poly-glucosides de décyle (GluC10 DP=1,3) sont dispersés à 15% massique dans un mélange eau/glycérol selon le protocole de l'exemple 1.

La proportion de GLUC10 Dp1,3 dans le mélange de tensioactif est notée R. Cette proportion varie de R=1 pour une solution contenant uniquement le Glu C10 Dp=1,3 à R=0 pour une solution contenant uniquement le XYL C10 DP=1,25. Une réplique de cryofracture est obtenue pour chacune des dispersions puis observée au MET. L'observation de vésicules multilamellaires est notée MLV, celle de phase lamellaire non enroulée est notée L et l'observation d'une phase micellaire est notée MC.

| R | 1 | 0,9 | 0,8 | 0,7 | 0,6 | 0,5 | 0, 4 | 0, 3 | 0,2 | 0,1 | 0 |
|---|---|---|---|---|---|---|---|---|---|---|---|
| Observations au MET | MC | MC | MC | MC | MC | MC | MC | L | MLV | MLV | MLV |

Cet exemple montre qu'il faudra au minimum 70% massique de pentosides d'alkyle (xylC10 DP=1,25) dans le mélange de tensioactif pour obtenir une phase lamellaire à la dilution de l'essai et au minimum 80% massique pour obtenir une dispersion homogène de vésicules multilamellaires.

Le polyglucoside de décyle et les mélanges contenant de 40 à 90% de ce polyglucoside ne forment pas de vésicules démontrant ainsi la spécificité des poly-xylosides d'alkyles à former de tels objets.

### Exemple 3

### Dispersion aqueuse de vésicules stable à basse température selon l'invention

Des mélanges de poly-xyloside de décyle (Xyl C10 DP=1,5) et de co-tensioactifs de différents HLB sont dispersés à 7,5% massique dans un mélange eau/glycérol selon le protocole décrit à l'exemple 1. Le rapport tensioactif sur co-tensioactif est égal à 10. Pour chacun des co-tensioactifs utilisés, les dispersions obtenues sont stockées à 5°C pendant 3 mois puis observées visuellement et au microscope optique (grossissement x10). Des répliques de cryofractures sont réalisées et observées au TEM sur l'ensemble des échantillons avant l'étude de stabilité à 5°C puis sur les échantillons stables au bout de 90 jours à 5°C.

Le tableau suivant rend compte des résultats obtenus en fonction de la HLB (Balance Hydrophile/lipophile) du co-tensioactif.

| HLB du co-tensioactif | Présence de MLV (t=0) | Présence de MLV (t=90j à 5°C) |
|---|---|---|
| 4,7 | Oui | - |
| 10,6 | Oui | - |
| 14,2 | Oui | Oui |
| 16,7 | Oui | Oui |

La présence de co-tensioactifs de haut HLB (>14) donne une bonne stabilité et une dispersion homogène de vésicules multilamellaires même après 3 mois à 5°C. L'utilisation de co-tensioactifs de bas HLB (<11) montre une altération des vésicules multilamellaires dans ces conditions de stockage.

### Exemple 4

### Dispersion huileuse de vésicules selon l'invention

Une première dispersion est réalisée en mélangeant à l'aide d'un agitateur mécanique à 500 tours/min et à 50°C, 45g de poly-xyloside d'octyle/décyle (XYL C8/C10 DP=1,3) avec 24g d'oléate de sorbitan (Radia 7125 de la société OLEON, HLB=4,7) et 31g d'eau. Après l'arrêt du chauffage, le mélange ainsi obtenu est laissé sous agitation mécanique jusqu'à ce qu'il soit à la température du laboratoire.

45g de ce mélange est ensuite dispersé dans 55g d'huile de paraffine (Marcol 82) sous agitation mécanique et à la température du laboratoire.

On obtient ainsi une dispersion homogène de phase cristal liquide dans l'huile et l'observation au microscope optique d'un échantillon placé entre deux polariseurs croisés montre la présence d'objets caractéristiques des vésicules.

### Exemple Comparatif 2

Des poly-xylosides de dodécyle (XylC12 DP=1,3) d'une part sont dispersés à 3,8% massique dans un mélange eau/glycérol selon le protocole de l'exemple 1.

D'autre part, des poly-xylosides de 2-butyl-octyle (Xyl Iso12 DP=1,3) sont dispersés à 15% massique dans un mélange eau/glycérol selon le protocole de l'exemple 1.

La première préparation donne un mélange hétérogène avec une sédimentation du système tensioactif. L'observation de la phase supérieure au MET ne montre pas la présence de vésicule.

La seconde préparation donne une solution légèrement turbide. L'observation de la solution au MET montre la présence d'une phase lamellaire non enroulée et l'absence totale de vésicule.

Cet exemple montre le xylosides d'alkyles aux degrés de polymérisation compris entre 1 et 1,5 t à chaînes longues et supérieures à 12 atomes de carbone ne sont pas aptes à former facilement des vésicules selon l'invention.

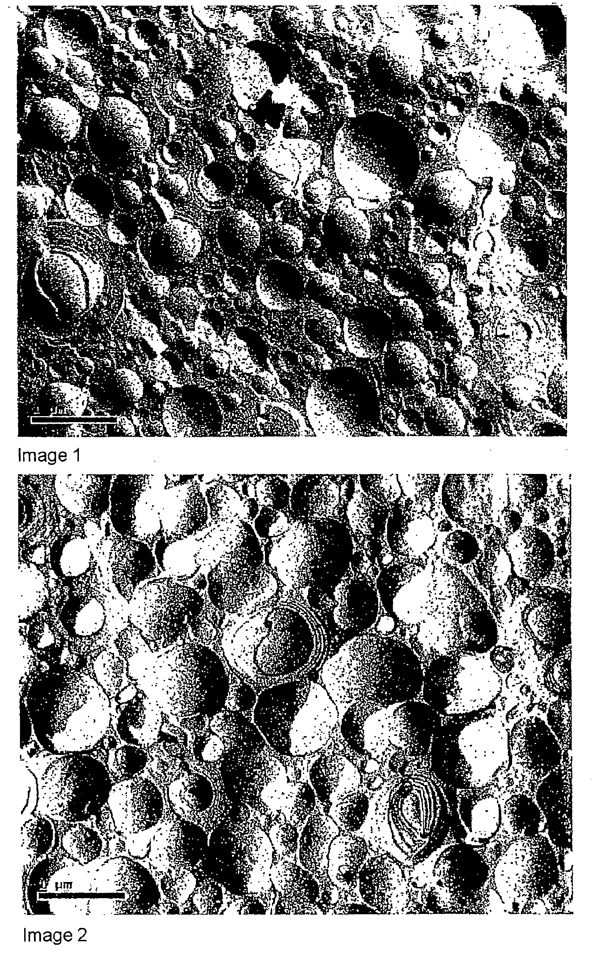

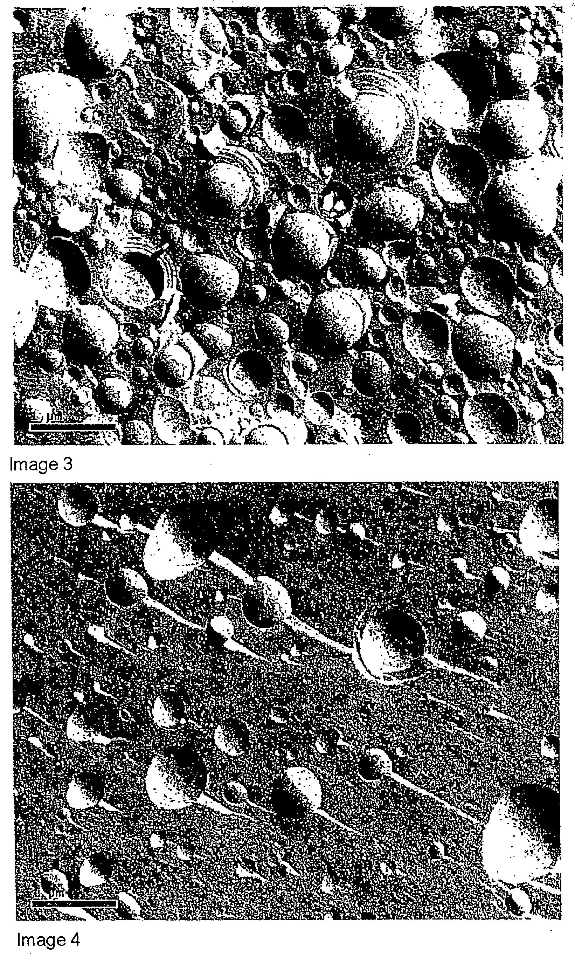

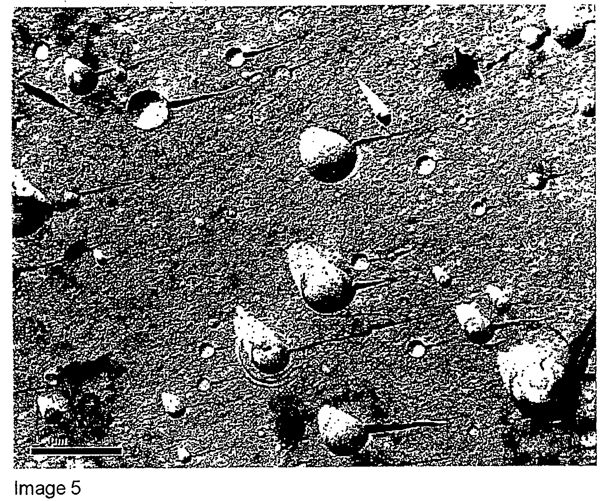

## Revendications

1. Procédé de préparation d'une dispersion de vésicules multilamellaires ou liposomes comprenant la dispersion d'un système tensioactif apte à former des vésicules ou liposomes dans une phase aqueuse, un solvant ou une huile, **caractérisé en ce que** ce système tensioactif contient en poids de 5 à 99%, et préférence de 70 à 90% en poids, d'au moins un polyglycoside d'alkyle de formule RO(X)n dans laquelle R est une chaîne alkyle linéaire ou ramifié, avec ou sans insaturation, et comportant 8 à 10 atomes de carbone, X est le reste de xylose, et n représente le degré moyen d'oligomérisation et est compris entre 1 et 3.

2. Procédé d'inclusion d'une ou plusieurs matières actives cosmétiques, pharmaceutiques, phytopharmaceutiques, de parfums, d'enzymes, de nutriments, d'oligo-éléments, de matériaux biologiques, d'huiles essentielles, d'agents aptes à oxyder ou dégrader des molécules organiques, dans des vésicules multilamellaires ou liposomes, consistant à mélanger lesdites matières actives brutes ou diluées dans une huile, un solvant ou dans l'eau avec un système tensioactif, puis à disperser ce mélange dans une phase aqueuse, un solvant ou une huile, **caractérisé en ce que** ce système tensioactif contient en poids de 5 à 99%, de préférence de 70 à 90% en poids, d'au moins un polyglycoside d'alkyle de formule RO(X)n dans laquelle R est une chaîne alkyle linéaire ou ramifié, avec ou sans insaturation, et comportant 8 à 10 atomes de carbone, X est le reste de xylose, et n représente le degrés moyen d'oligomérisation et est compris entre 1 et 3.

3. Système tensioactif **caractérisé en ce qu'**il comprend en poids :
- de 5 à 99%, de préférence de 30 à 90%, de polyglycoside d'alkyle de formule RO(X)n, dans laquelle R est une chaîne alkyle linéaire ou ramifiée, avec ou sans insaturation, et comportant 8 à 10 atomes de carbone, X étant le reste de xylose, et n représentant le degré moyen d'oligomérisation est compris entre 1 et 3, de préférence entre 1 et 1,5 ;
- de 0,1 à 70% d'un ou plusieurs co-tensioactifs ayant une HLB au moins égale à 10 ; et
- de 0,1 à 70% d'eau.

4. Système tensioactif suivant la revendication 3, **caractérisé en ce qu'**il comprend en poids :
- de 0 à 70% d'un hydrotrope ou co-solvant choisi parmi les alcools linéaires ou ramifiés de C1 à C20, les éthers de glycols, les glycosides d'amyle, de butyle, d'hexyle, de 2-éthyl-hexyle, d'octyle, d'iso-nonyle, d'iso-décyle, d'octyle-décyle, le glycérol et les dérivés partiels de glycérol, les terpinéols, les esters d'acides organiques comme l'acide acétique, formique, lactique, succinique, tartrique, glutarique, glutamique, adipique, laurique, oléique.

5. Système tensioactif **caractérisé en ce qu'**il comprend en poids :
- de 5 à 99%, de préférence de 30 à 90%, de polyglycoside d'alkyle de formule RO(X)n, dans laquelle R est une chaîne alkyle linéaire ou ramifiée avec ou sans insaturation, et comportant 8 à 10 atomes de carbone, X étant le reste de xylose, et n représentant le degré moyen d'oligomérisation est compris entre 1 et 3, de préférence entre 1 et 1,5 ;
- de 0,1 à 70% d'un ou plusieurs co-tensioactifs ayant une HLB inférieure à 10 ; et
- de 0,1 à 70% d'eau.

6. Système tensioactif suivant la revendication 5, **caractérisé en ce qu'**il comprend en poids :
- de 0,1 à 70% d'un hydrotrope ou co-solvant choisi parmi les alcools linéaires ou ramifiés de C1 à C20, les éthers de glycols, les glycosides d'amyle, de butyle, d'hexyle, de 2-éthyl-hexyle, d'octyle, d'iso-nonyle, d'iso-décyle, d'octyle-décyle, le glycérol et les dérivés partiels de glycérol, les terpinéols, les esters d'acides organiques comme l'acide acétique, formique, lactique, succinique, tartrique, glutarique, glutamique, adipique, laurique, oléique.

7. Système suivant l'une des revendications 3 à 6, **caractérisé en ce qu'**il est sous la forme de vésicules ou liposomes qui contiennent de 1 à 99% en poids par rapport au poids total des vésicules ou liposomes, de préférence de 10 à 95% et de préférence encore de 30 à 90%, d'une ou plusieurs matières actives cosmétiques, pharmaceutiques, phytopharmaceutiques, de parfums, d'enzymes, de nutriments, d'oligo-éléments, de matériaux biologiques, d'huiles essentielles, d'agents aptes à oxyder ou dégrader des molécules organiques, brutes ou diluées dans une huile, un solvant ou dans l'eau.

8. Système suivant la revendication 7, **caractérisé en ce qu'**il est sous la forme d'une émulsion huile dans eau, solution aqueuse ou hydro-alcoolique, gel aqueux ou hydro-alcoolique qui contient de 0,01 à 90% en poids, de préférence de 0,1 à 50% et de préférence encore de 1 à 20% en poids de vésicules ou liposomes.

9. Système suivant la revendication 7, **caractérisé en ce qu'**il est sous la forme d'une émulsion eau dans huile, solvants ou mélanges de solvants, microémulsion, qui contient de 0,01 à 90% en poids, de préférence de 0,1 à 50% en poids et de préférence encore de 1 à 10% en poids de vésicules ou liposomes.

10. Utilisation d'une dispersion de vésicules ou liposomes selon l'une quelconque des revendications 8 ou 9 pour la formulation de produits cosmétiques, pharmaceutiques, phytopharmaceutiques, détergents, pour le traitement des eaux ou la dépollution des sols.
